# EUROPEAN PATENT APPLICATION

(11) **EP 4 564 273 A1**
(43) Date of publication of application: **04.06.2025**
(21) Application number: 23846525.6
(22) Date of filing: 25.07.2023
(51) Int. Cl.: G06Q 40/08, A01K 29/00, C12Q 1/04, C12Q 1/6869

(54) **INSURANCE PREMIUM CALCULATION SYSTEM, BEAUTY LEVEL ESTIMATION SYSTEM, AND OVERALL HEALTH ESTIMATION SYSTEM**

(30) Priority: 29.07.2022 JP 2022121710; 02.11.2022 JP 2022176765; 29.11.2022 JP 2022190464
(71) Applicant: Anicom Holdings, Inc., Shinjuku-ku Tokyo 160-0023 (JP)
(72) Inventor: TAKAHASHI Hiroyuki, Tokyo 160-0023 (JP); MATSUMOTO Kengo, Tokyo 160-0023 (JP); AMANO Mihoko, Tokyo 160-0023 (JP); TSUBOUCHI Chiharu, Tokyo 160-0023 (JP); TANAKA Hideomi, Tokyo 160-0023 (JP); ISHIZUKA Haruna, Tokyo 160-0023 (JP)
(74) Representative: Gill Jennings & Every LLP
(86) International application number: PCT/JP2023/027236
(87) International publication number: WO 2024/024795

(57) **Abstract**

An objective of the present invention is to provide a beauty level estimation system and a beauty level estimation method for calculating a beauty level of a pet animal kept by a user based on a correlation between diversity data related to diversity of intestinal microbiota of an animal and a beauty level. The beauty level estimation system includes: an acquisition unit that acquires diversity data of intestinal microbiota of a pet animal kept by a user; and a calculation unit that calculates a beauty level of the pet animal kept by the user based on the diversity data of the intestinal microbiota and a correlation between diversity data of intestinal microbiota of a pet animal and a beauty level.

## Description

### TECHNICAL FIELD

The present invention relates to an insurance premium calculation system, a beauty level estimation system, and an overall health estimation system, as well as, an insurance premium calculation method, a beauty level estimation method, and an overall health estimation method. More particularly, the present invention relates to: an insurance premium calculation system and an insurance premium calculation method for calculating an insurance premium suitable for a pet animal kept by its owner (hereinafter, sometimes referred to as a "user") based on a correlation between data on the diversity of animal intestinal microbiota (hereinafter referred to as "diversity data") and an insurance risk and/or a beauty level; a beauty level estimation system and a beauty level estimation method for calculating a beauty level; and an overall health estimation system and an overall health estimation method for calculating an overall health level.

### BACKGROUND ART

Pet animals such as dogs, cats, and rabbits, and domestic animals such as cows and pigs are irreplaceable to humans. While the average life expectancy of animals kept by humans has increased significantly in recent years, animals are more likely to suffer from some kind of injury or illness during their lifetime, and the increase in medical costs borne by their owners has become a problem.

To maintain the health of animals, it is important to control their physical conditions through daily diet, exercise, and the like, and to respond quickly to any problems. However, animals are not able to complain about their physical discomfort in their own language, and it is only when symptoms develop and some externally observable signs occur that owners become aware that their animals are suffering from an illness. In addition, when the musculoskeletal system is weakened due to calcium or vitamin deficiencies, external signs are rare, and the abnormality of an animal is noticed only when it suffers a fracture or other injury.

Furthermore, health is closely related to beauty, and healthy animals, including humans, have excellent fur or hair gloss and are of an adequate weight (neither thin nor fat). However, it is difficult to determine the superiority or inferiority of fur gloss or the like because animal owners rarely closely compare the animals they keep with those they do not keep. In particular, it is extremely difficult for animal owners to determine the superiority or inferiority of the fur gloss or the like of their pets, especially in the case of dogs, cats, and other animals whose fur gloss and physique vary greatly from one breed to another. If the beauty level of pet animals can be easily determined, it is expected that users will have an opportunity to review the dietary habits and other aspects of the lives of their pet animals.

Therefore, there is a need for a means of estimating an insurance premium and other financial burdens borne by an animal owner, as well as, a means of presenting the beauty level of an animal, such as fur gloss, in a simple manner.

Patent Document 1 discloses an intestinal microbiota regulating or improving composition that effectively regulates or improves the intestinal microbiota by increasing bacteria of the Bacteroidetes phylum and decreasing bacteria of the Firmicutes phylum in the intestinal microbiota. However, there is no disclosure of a method for presenting an insurance risk or beauty level based on data concerning the intestinal microbiota of animals.

### RELATED ART DOCUMENT

### PATENT DOCUMENT

[Patent Document 1] WO2017/094892

### SUMMARY OF THE INVENTION

### PROBLEMS TO BE SOLVED BY THE INVENTION

Therefore, an objective of the present invention is to provide a means of estimating an insurance premium and other financial burdens to be borne by an animal owner, as well as, a method of presenting the beauty level of an animal, such as fur gloss, in a simple manner.

### MEANS FOR SOLVING THE PROBLEMS

As a result of analyzing and examining a huge amount of data on the diversity of animals with pet insurance and the presence or absence of an insurance claim record, that is, the presence or absence of an injury or illness, of such animals, the inventors have found that it is possible to calculate an insurance premium suitable for each animal and predict a financial burden (medical costs) to be borne by a user, using the diversity data of the intestinal microbiota of the animal. In addition, the inventors analyzed and examined the diversity data of animals with pet insurance and their beauty levels regarding the superiority or inferiority of fur gloss and the like of the animals, and found that the diversity data of animal intestinal microbiota can be used to predict the beauty level of each animal.

Furthermore, the inventors found that an overall health level can be predicted by combining a beauty level and an insurance risk, as beauty is a reasonable indicator for determining whether an animal is not sick but not healthy, or so-called unwell.

In other words, the present invention can be described as in the following [1] to [27].
[1] An insurance premium calculation system including: an acquisition unit that acquires diversity data of intestinal microbiota of a pet animal kept by a user; and a calculation unit that calculates an insurance risk of the pet animal kept by the user based on the diversity data of the intestinal microbiota and a correlation between diversity data of intestinal microbiota of a pet animal and an insurance risk.
[2] The insurance premium calculation system according to [1], wherein the acquisition unit further acquires basic information of the pet animal kept by the user.
[3] The insurance premium calculation system according to [1] or [2], further including: a setting unit that sets a correlation between diversity data of intestinal microbiota of a pet animal and an insurance risk, wherein the calculation unit calculates an insurance risk of the pet animal kept by the user based on the diversity data of the intestinal microbiota and the correlation set by the setting unit.
[4] The insurance premium calculation system according to [1], wherein the correlation between the diversity data of the intestinal microbiota of the pet animal and the insurance risk is a negative correlation.
[5] The insurance premium calculation system according to [1], wherein the diversity data of the intestinal microbiota of the pet animal is represented by a Shannon index.
[6] The insurance premium calculation system according to [2], wherein the calculation unit calculates an insurance premium by adjusting an insurance premium calculated based on the basic information of the pet animal, according to an insurance risk derived using the diversity data of the intestinal microbiota of the pet animal.
[7] The insurance premium calculation system according to [2], wherein the calculation unit calculates an insurance premium based on a value obtained by adjusting a predicted medical cost calculated based on the basic information of the pet animal, according to an insurance risk derived using the diversity data of the intestinal microbiota.
[8] The insurance premium calculation system according to [2], wherein the calculation unit calculates an insurance premium based on a value obtained by adjusting a predicted medical cost calculated based on the basic information of the pet animal by a factor of 0.5 to 2.0, according to an insurance risk derived using the diversity data of the intestinal microbiota.
[9] The insurance premium calculation system according to [2], wherein the basic information of the pet animal is at least breed and age.
[10] The insurance premium calculation system according to [1] or [2], further including: a prediction calculation unit that adjusts an insurance risk calculated by the calculation unit, based on information of food consumed by the pet animal kept by the user.
[11] An insurance premium calculation method including the steps of: acquiring diversity data of intestinal microbiota; and calculating, by a computer, an insurance risk of a pet animal kept by a user based on the diversity data of the pet animal kept by the user and a correlation.
[12] The insurance premium calculation method according to [11], further including the step of: acquiring basic information of the pet animal kept by the user.
[13] The insurance premium calculation method according to [12], wherein the basic information of the pet animal is at least breed and age.
[14] The insurance premium calculation method according to [11] or [12], further including the step of: adjusting, based on information of food that the pet animal kept by the user eats, an insurance risk derived in the step of calculating the insurance risk of the pet animal kept by the user based on the diversity data of the pet animal kept by the user and the correlation.
[15] A financial burden predicting method including the steps of: acquiring basic information of a pet animal kept by a user and diversity data of intestinal microbiota; setting a correlation between diversity data of intestinal microbiota of a pet animal and a burden risk; and calculating, by a computer, a burden risk of the pet animal kept by the user based on the diversity data of the pet animal kept by the user and the correlation.
[16] A beauty level estimation system including: an acquisition unit that acquires diversity data of intestinal microbiota of a pet animal kept by a user; and a calculation unit that calculates a beauty level of the pet animal kept by the user based on the diversity data of the intestinal microbiota of the pet animal and a correlation between diversity data of intestinal microbiota of a pet animal and a beauty level.
[17] The beauty level estimation system according to [16], wherein the acquisition unit further acquires basic information of the pet animal kept by the user.
[18] The beauty level estimation system according to [16] or [17], wherein the beauty level is a level related to fur gloss or a level related to a body shape.
[19] The beauty level estimation system according to [16], wherein the diversity data of the intestinal microbiota of the pet animal is represented by a Shannon Index.
[20] The beauty level estimation system according to [17], wherein the calculation unit calculates a beauty level by adjusting a beauty level calculated based on the basic information of the pet animal according to a beauty variation factor derived using the diversity data of the intestinal microbiota of the pet animal.
[21] The beauty level estimation system according to [17], wherein the basic information of the pet animal is at least breed and age.
[22] A beauty level estimation system, including: an acquisition unit that acquires diversity data of intestinal microbiota of a pet animal kept by a user; and a calculation unit that calculates a beauty level of the pet animal from the diversity data of the intestinal bacteria acquired by the acquisition unit using a learned model, wherein the learned model is a learned model that has learned a relationship between diversity data of intestinal microbiota of a pet animal and a beauty level of the pet animal.
[23] An overall health estimation system including: an acquisition unit that acquires diversity data of intestinal microbiota of a pet animal kept by a user; a calculation unit that calculates a beauty level of the pet animal kept by the user based on the diversity data of the intestinal microbiota and a correlation between diversity data of intestinal microbiota of a pet animal and a beauty level; a risk calculation unit that calculates an insurance risk of the pet animal kept by the user based on the diversity data of the intestinal microbiota and a correlation between diversity data of intestinal microbiota of a pet animal and an insurance risk; and a health level calculation unit that calculates a health level based on the beauty level and the insurance risk.
[24] A beauty level estimation method including, in the following order, the steps of: acquiring diversity data of intestinal microbiota of a pet animal; and calculating, by a computer, a beauty level of the pet animal kept by a user based on the diversity data and a correlation between diversity data of intestinal microbiota of a pet animal and a beauty level.
[25] The beauty level estimation method according to [24], further including the step of acquiring basic information of the pet animal kept by the user.
[26] The beauty level estimation method according to [25], wherein the basic information of the pet animal is at least breed and age.
[27] The beauty level estimation method according to [25] or [26], further including the step of: adjusting, based on information of food that the pet kept by the user eats, a beauty level derived in the step of calculating the beauty level of the pet animal kept by the user based on the diversity data of the pet animal kept by the user and the correlation between the diversity data of the intestinal microbiota of the pet animal and the beauty level.

### EFFECTS OF THE INVENTION

The present invention makes it possible to provide a beauty level estimation system, a beauty level estimation method, an overall health estimation system, and an overall health estimation method that can present the beauty level of a pet animal in a simple manner.

### BRIEF DESCRIPTION OF THE DRAWINGS

FIG. 1 is a schematic diagram of an insurance premium calculation system.
FIG. 2 is a diagram illustrating an insurance premium calculation method.
FIG. 3 is a model diagram illustrating a correlation between diversity data (diversity index) and insurance risk.
FIG. 4 is a diagram illustrating a correlation between diversity data and insurance risk.
FIG. 5 is a diagram illustrating the results of correlation analysis.
FIG. 6 is a diagram illustrating a correlation between diversity data and insurance risk.
FIG. 7 is a diagram illustrating a correlation between diversity data and insurance risk.
FIG. 8 is a diagram illustrating a correlation between diversity data and insurance risk.
FIG. 9 is a diagram illustrating a correlation between diversity data and insurance risk.
FIG. 10 is a diagram illustrating a correlation between diversity data and insurance risk.
FIG. 11 is a diagram illustrating a correlation between diversity data and insurance risk.
FIG. 12 is a diagram illustrating a correlation between diversity data and insurance risk.
FIG. 13 is a diagram illustrating a correlation between diversity data and insurance risk.
FIG. 14 is a diagram illustrating a correlation between diversity data and insurance risk.
FIG. 15 is a diagram illustrating a correlation between diversity data and insurance risk;
FIG. 16 is a diagram illustrating a correlation between diversity data and insurance risk.
FIG. 17 is a diagram illustrating the results of correlation analysis.
FIG. 18 is a diagram illustrating a correlation between diversity data and insurance risk.
FIG. 19 is a diagram illustrating a correlation between diversity data and insurance risk.
FIG. 20 is a diagram illustrating a correlation between diversity data and insurance risk.
FIG. 21 is a diagram illustrating a correlation between diversity data and insurance risk.
FIG. 22 is a diagram illustrating a correlation between diversity data and insurance risk.
FIG. 23 is a diagram illustrating a relationship between the number of foods and diversity data.
FIG. 24 is a diagram illustrating a relationship between the number of foods and diversity data.
FIG. 25 is a schematic diagram of a beauty level estimation system.
FIG. 26 is a diagram illustrating a beauty level estimation method.
FIG. 27 is a diagram illustrating a correlation between diversity data and beauty level (fur gloss).
FIG. 28 is a diagram illustrating a correlation between diversity data and beauty level (fur gloss).
FIG. 29 is a diagram illustrating a correlation between diversity data and beauty level (standard body weight).

### MODE FOR CARRYING OUT THE INVENTION

An insurance premium calculation system is first described. The insurance premium calculation system, together with the beauty level estimation system described below, is a necessary component for establishing an overall health estimation system.

### [Insurance premium calculation system]

The insurance premium calculation system of the present invention includes: an acquisition unit that acquires diversity data of intestinal microbiota; and a calculation unit that calculates an insurance risk of a pet animal kept by a user based on the diversity data of the pet animal kept by the user and the correlation. Preferably, in the insurance premium calculation system of the present invention, the acquisition unit further acquires basic information of the pet animal kept by the user. It is also preferable that the insurance premium calculation system of the present invention further includes a setting unit that sets a correlation between the diversity data of the intestinal microbiota of a pet animal and an insurance risk.

### [System overview]

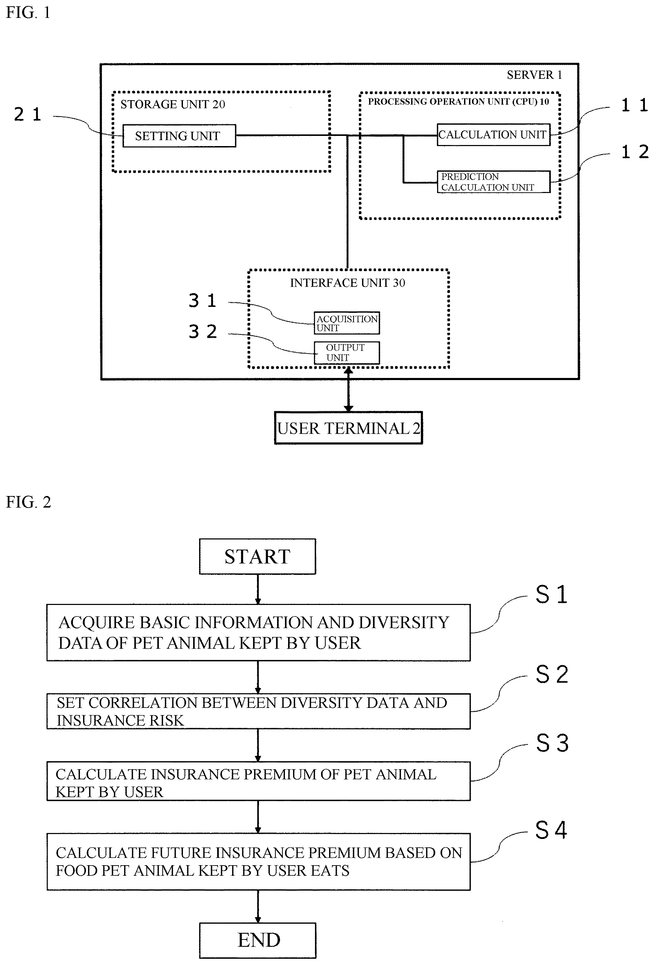

FIG. 1 is a schematic diagram of an insurance premium calculation system according to an embodiment of the present disclosure. As illustrated in FIG. 1, the insurance premium calculation system according to the present embodiment includes a server 1 and a user terminal 2. The server 1 and the user terminal 2 are connected via a network. The server 1 includes a processing operation unit (CPU) 10, a storage unit 20, and an interface unit 30. Users in the present invention include pet owners, as well as, agents, breeders, and the like. Pet animals include dogs, cats, rabbits, birds, reptiles, and amphibians while dogs, cats, and rabbits are preferred.

Furthermore, the processing operation unit (CPU) 10 includes a calculation unit 11 and a prediction calculation unit 12; the storage unit 20 includes at least a setting unit 21; and the interface unit 30 includes an acquisition unit 31 and an output unit 32. The setting unit 21 may be configured to store a formula, a function, a table or software pertaining to the correlation between the diversity data of the intestinal microbiota and the insurance risk. In such a configuration, the calculation unit 11 can retrieve the formula, function, table or software stored in the setting unit 21 based on the basic information of a pet animal kept by a user, and calculate an insurance risk based on the diversity data of the pet animal kept by the user. The insurance risk is an indicator indicating the probability of the occurrence of an event that is subject to insurance payment. The event that is subject to insurance payment is an injury or illness in the context of animal insurance. The insurance risk is reflected in an insurance premium, which increases with higher risk and decreases with lower risk. The insurance premium is the amount of money paid by the user to the insurance company, and the insurance payment is the amount of money paid by the insurance company to the user upon the occurrence of the event.

Here, the calculation unit 11 calculates an insurance risk of a pet animal kept by a user based on the diversity data of the pet animal kept by the user and the correlation mentioned above. The embodiment illustrated in FIG. 1 further includes a prediction calculation unit 12. The prediction calculation unit 12 predicts a future insurance risk of a pet animal kept by a user based on information of the food fed to the pet animal. For example, an insurance risk is calculated by adjusting an insurance risk once calculated by the calculation unit 11, based on the food information.

The setting unit 21 also sets a correlation between the diversity data of the intestinal microbiota of a pet animal and an insurance risk.

In addition, the acquisition unit 31 acquires the diversity data of the intestinal microbiota and, preferably, further acquires basic information of the pet animal kept by the user, and the output unit 32 sends the insurance risk, insurance premium, and copayment calculated by the calculation unit 11 to the user.

### [Diversity data]

Diversity data are data related to the diversity of bacteria in the intestinal microbiota of an animal. A large diversity of the intestinal microbiota means that the intestinal microbiota contains a wide variety of bacteria evenly over a wide area. Although there are several types of indicators for diversity data, that is, diversity indices, any of the known ones may be used in the present invention. The diversity indices include the Shannon-Wiener diversity index (hereinafter, abbreviated as "Shannon index"), the Simpson index, the number of unique sequences detected by sequencing (amplicon sequence variant: ASV), the number of operational taxonomic units (OTUs), Faith's PD, and Pielou's evenness.

### [Measurement of diversity data]

The diversity data of the intestinal microbiota can be measured using known metagenomic and microbiota analysis methods such as amplicon sequencing and shotgun sequencing using NGS and other sequencers. For example, a sample such as feces is collected from an animal, and the DNA and RNA base sequence information of any organisms contained in the sample is analyzed using a next-generation sequencer to identify the organisms contained in the sample. Preferably, all or part of the 16S rRNA genes contained in the sample are amplified as necessary, and sequenced, and the obtained sequence is analyzed using software to obtain composition data of the bacteria in the sample.

An example of 16S rRNA gene amplicon analysis (16S metagenomic analysis) using a next generation sequencer (NGS) is described below in detail. First, DNA is extracted from a sample using a DNA extraction reagent, and the 16S rRNA genes are amplified from the extracted DNA by PCR. The amplified DNA fragments are then comprehensively sequenced using NGS to remove low quality reads and chimeric sequences, and then the sequences are clustered together for operational taxonomic unit (OTU) analysis. OTU is an operational taxonomic unit that allows sequences with more than a certain degree of similarity (for example, 96-97% homology) to be treated as if they belong to a single bacterial species. Therefore, the number of OTUs represents the number of bacterial species constituting the flora, and the number of reads belonging to the same OTU is considered to represent the relative abundance of that species. By selecting representative sequences from the number of reads belonging to each OTU, the names of families and genera can be identified by database search. In this way, the number of bacterial species belonging to a particular family or phylum can be determined. Analysis by amplicon sequence variant (ASV) is also possible; ASV is created after removing erroneous sequences generated during PCR and sequencing, and thus sequence variations in single nucleotide units can be distinguished, allowing for finer identification. The insurance premium calculation system of the present invention may use pre-measured diversity data, or it may receive a fecal sample from a user, measure the diversity data, and use that diversity data. When using pre-measured diversity data, a user sends a fecal sample to, for example, an enterobacteria analyst and request to measure the intestinal microbiota, then, receives the diversity data from the analyst who undertook the request. The user can send the diversity data received in this way to the insurance premium calculation system through a user terminal 2. The system may instead be configured in such a way that the analyst who undertook the request sends the diversity data to the insurance premium calculation system of the present invention on behalf of the user.

Next, the technical features of the present invention are described in detail.

### [Insurance risk calculation based on diversity data]

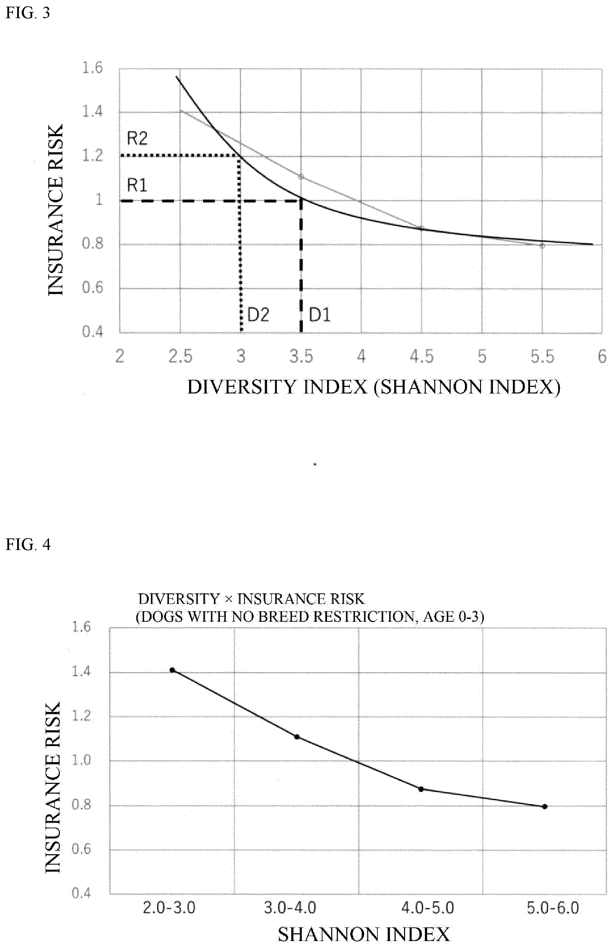

The following describes an example of the insurance risk calculation method based on diversity data with reference to FIG. 3 (model diagram). FIG. 3 is a graph for illustrating an example of an insurance risk calculation method based on diversity data according to the present embodiment. The vertical axis of this graph is the insurance risk and the horizontal axis is the diversity data. Specifically, when the diversity index (Shannon index) is 3.5 (D1), the insurance risk (R1) is 1. This means that the probability that a pet animal with a diversity index of 3.5 will suffer an injury or illness is the same as the average of all pet animals. In other words, if the average treatment cost for all pet animals is 5,000 yen/month, it means that the treatment cost for a pet animal with a diversity index of 3.5 would be also 5,000 yen/month (the copayment borne by the pet owner). On the other hand, when the diversity index is 3 (D2), the insurance risk (R2) is 1.2. This means that the treatment cost for a pet animal with a diversity index of 3.5 would be 6,000 yen.

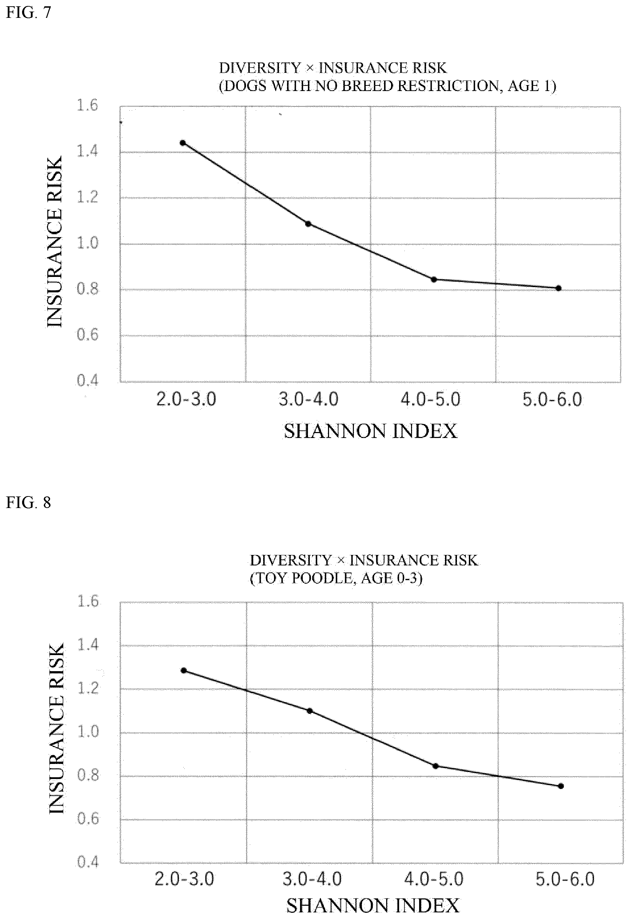

More specifically, FIG. 8 illustrates the insurance risk of toy poodles aged 0 to 3 years, with the insurance risk on the vertical axis and the diversity index (Shannon index) on the horizontal axis. For statistical analysis, the diversity index is divided into four categories ((1) 2.0 or more and less than 3.0, (2) 3.0 or more and less than 4.0, (3) 4.0 or more and less than 5.0, and (4) 5.0 or more and less than 6.0). As illustrated in FIG. 8, a correlation exists between the diversity data and the insurance risk for toy poodles aged 0 to 3 years.

### [Setting unit]

The server or storage unit may be equipped with a setting unit that sets a correlation between the diversity data of the intestinal microbiota of a pet animal and an insurance risk. The correlation herein refers to information indicating the correspondence between the degree of diversity data and the degree of insurance risk. The correlation may be viewed as a model (a function) with diversity data as input and insurance risk as output. For example, a correlation is set by statistically processing the diversity data of a plurality of pet animals and the costs incurred for injury, illness, and treatment of the pet animals. In principle, the pet animals used to set the correlation and the pet animal kept by a user that is subject to insurance risk calculation are different individuals.

The correlation may instead be set based on the basic information of a pet animal kept by a user. The basic information may include age and breed. For example, the server sets a correlation by statistically processing the diversity data of the pet animals that have similar basic information to that of a pet animal kept by a user and the costs incurred for the injury, illness, and treatment of the pet animals. The server may pre-establish a correlation for each category of basic information (for example, age, breed) and set a correlation for the category corresponding to the basic information of the pet animal kept by the user. The setting unit does not need to set a correlation each time the system runs, but may be configured in such a way that the correlation once set by the setting unit is continuously used by the calculation unit thereafter. The configuration may also be such that a correlation is set each time data pertaining to insurance risk and diversity data are updated.

Setting a correlation between diversity data and an insurance risk enables calculation of an insurance risk and an insurance premium based on the diversity data of a pet animal kept by a user.

### [Insurance premium calculation method]

FIG. 2 is a diagram illustrating an overview of the insurance premium calculation method according to an embodiment of the present disclosure.

As illustrated in FIG. 2, the insurance premium calculation method in the present embodiment includes the following steps in the following order: Step S1 for acquiring basic information of a pet animal kept by a user and diversity data of the intestinal microbiota; Step S2 for setting a correlation between the diversity data of the intestinal microbiota of a pet animal and an insurance risk; Step S3 for calculating an insurance risk of the pet animal kept by the user based on the diversity data of the pet animal kept by the user and the correlation.

Here, it is preferable to further include a step for acquiring basic information of the pet animal kept by the user, and for setting a correlation based on the basic information of the pet animal kept by the user. Setting a correlation in accordance with the basic information of a pet animal kept by a user enables calculation of an insurance premium more suitable for the pet animal. It is more preferable to first calculate a provisional insurance premium based on the basic information, and then adjust the provisional insurance premium by taking into account an insurance risk based on the diversity data to calculate a final insurance premium. When the provisional insurance premium based on the basic information is adjusted by taking into account the insurance risk based on the diversity data in such a way, it is preferable to adjust the provisional insurance premium by a factor of 0.5 to 2.0. The inventors have found that the insurance risk of an individual pet animal varies between 0.5 and 2.0 times the average depending on the diversity data. Alternatively, a predicted medical cost may be once calculated based on the basic information, and an insurance premium may be calculated based on a medical cost obtained by adjusting the medical cost according to an insurance risk derived from the diversity data.

Furthermore, the system may include the step for predicting a future insurance risk of the pet animal kept by the user based on information of the food that the pet animal kept by the user eats. Since diversity increases depending on the food that the pet animal eats, the future insurance premium can be calculated by taking the food information into account.

Note that the insurance premium is generally the medical cost of an animal multiplied by the contribution rate of the insurance company, plus the commissions, operating expenses and other costs of the insurance company. Therefore, it is possible to predict the financial burden (medical cost) to be borne by a user in a similar way to the calculation of an insurance premium by the insurance premium calculation system.

The following describes a reference example of the insurance premium calculation system.

### [Example 1]

### (Selection of dogs)

The insurance risks of approximately 110,000 individuals whose diversity indices of intestinal microbiota were measured from fecal samples within the policy period (one year) were investigated based on insurance claims during the same period. The presence or absence of an injury or illness prior to fecal sample collection was not taken into account, so individuals with an injury or illness prior to sample collection were included.

### (DNA extraction from fecal samples)

A fecal sample was collected from a dog and DNA was extracted as follows.

The dog owner collected a fecal sample from their dog using a fecal collection kit. The fecal sample was received and suspended in fixative solution (10% EtOH, 1.07% NH4Cl, 5 mM EDTA, 0.09% NaN3).

Next, 200 uL of the fecal suspension and 810 uL of Lysis buffer (containing 224 ug/mL Protenase K) were added to a bead tube, and bead disruption (6,000 rpm; 20 sec disruption, 30 sec interval, 20 sec disruption) was performed in a bead homogenizer. The specimen was then treated with Protenase K by placing it on a heat block at 70 °C for 10 minutes, followed by inactivation of Protenase K by placing it on a heat block at 95°C for 5 minutes. The lysed specimen was subjected to automated DNA extraction using chemagic 360 (PerkinElmer) with the protocol for chemagic kit stool to obtain 100 uL of DNA extract.

### (16S RNA metagenomic sequencing analysis)

16S metagenomic sequencing analysis was performed using a modification of the Illumina 16S Metagenomic Sequencing Library Preparation (version 15044223 B). First, a 460 bp region containing the variable region V3-V4 of the 16S rRNA gene was amplified by PCR using universal primers (Illumina_16S_341F and Illumina_16S_805RPCR). The PCR reaction solution was prepared by mixing 10 uL of the DNA extract, 0.05 uL of each primer (100 uM), 12.5 uL of 2x KAPA HiFi Hot-Start ReadyMix (F. Hoffmann-La Roche, Switzerland), and 2.4 uL of PCR grade water. The PCR involved heat denaturation at 95°C for 3 min, followed by 30 cycles of 95°C for 30 sec, 55°C for 30 sec, and 72°C for 30 sec, and finally an elongation reaction at 72°C for 5 min. The amplified product was purified using magnetic beads and eluted with 50 uL of Buffer EB (QIAGEN, Germany). The purified amplified product was subjected to PCR using Nextera XT Index Kit v2 (Illumina, CA, US) and indexed. The PCR reaction solution was prepared by mixing 2.5 uL of the amplified product, 2.5 uL of each primer, 12.5 uL of 2x KAPA HiFi Hot-Start ReadyMix, and 5 uL of PCR grade water. The PCR involved heat denaturation at 95°C for 3 min, followed by 12 cycles of 95°C for 30 sec, 55°C for 30 sec, and 72°C for 30 sec, and finally an elongation reaction at 72°C for 5 min. The amplified product after index addition was purified using magnetic beads and eluted with 80-105 uL of Buffer EB. The concentration of each amplified product was measured with a NanoPhotometer (Implen, CA, US), prepared to 1.4 nM, and mixed in equal volumes to make a library for sequencing. The DNA concentrations of the sequencing library and the size of the amplified product were confirmed by electrophoresis, which was analyzed by MiSeq. Paired-end sequencing of 2 x 300 bp was performed using MiSeq Reagent Kit V3. The obtained sequences were analyzed by QIIME2 analysis software to obtain bacterial composition data.

The sequences of the universal primers used above are as follows. These universal primers can be purchased commercially.
Illumina_16S_341F 
5’-
TCGTCGGCAGCGTCAGATGTGTATAAGAGAGACAGCCTACGGGGNGGCWGCAG- 3’
llumina_16S_805R

5’-
GTCTCGTGGGCTCGGAGATGTGTATAAGAGACAGGACTACHVGGGTATCTAATCC-
3’

According to the above method, composition data of the intestinal microbiota were obtained for all breeds of dogs aged 0 years and older and 3 years and younger, and the diversity indices (Shannon index and ASV index) were measured using QIIME2. The number of valid individuals was 105335 for the Shannon index and 105677 for the ASV index, excluding outliers.

### (Confirmation of injury/illness and insurance claim)

For all the dog breeds, the inventors investigated whether or not an insurance claim was filed within the policy period. As noted above, the presence or absence of an injury or illness prior to fecal sample collection was not taken into account, so individuals with an injury or illness prior to sample collection were included.

### (Correlation chart)

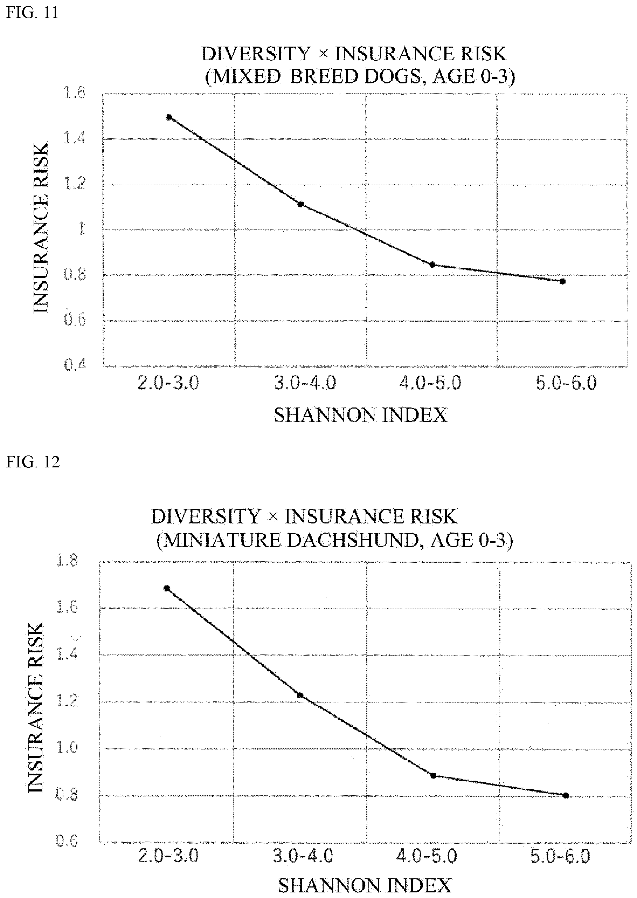

The correlations between the diversity data and the insurance risk obtained by the above experiment are illustrated in FIG. 4 and FIGS. 6 to 13. The vertical axis is the insurance risk and the horizontal axis is the diversity index.

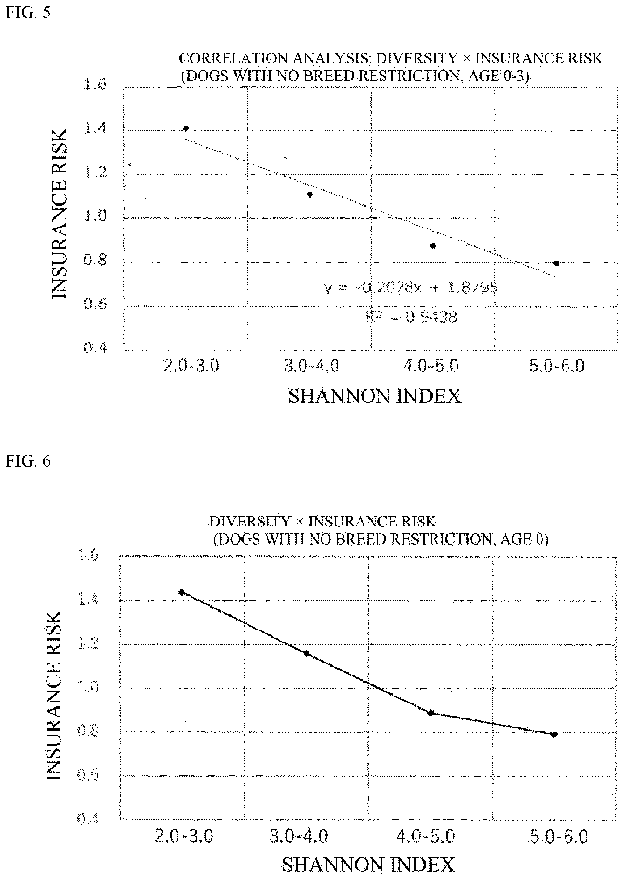

FIG. 5 illustrates the result of the correlation analysis of FIG. 4 (dogs with no breed restriction, aged 0 to 3), which reveals that a correlation is established between the diversity data and the insurance risk.
Correlation coefficient: -0.97 (negative correlation)
Approximate formula: y = -0.2078x + 1.8795
Coefficient of determination: R² = 0.9438

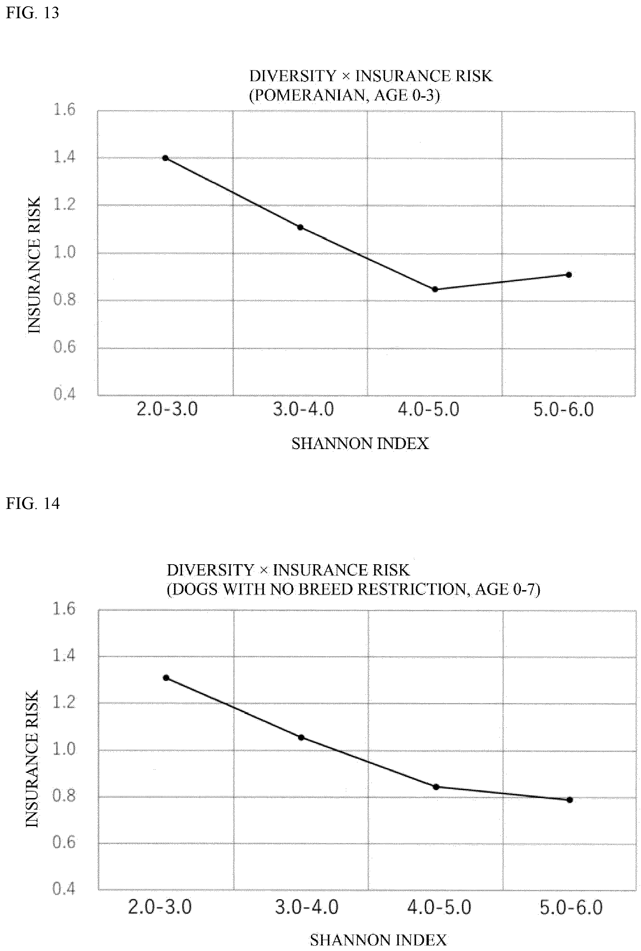

To explain a correlation can be established for dogs other than those older than 0 years and older and 3 years and younger, the inventors investigated correlations under other conditions (FIGS. 14 to 16).

FIG. 14 is a correlation chart between diversity data and an insurance risk for all dog breeds (136,033 individuals) aged 0 to 7 years. This result reveals that there is a correlation between diversity data and an insurance risk not only for dogs aged 0 years and older and 3 years and younger, but also for 4 years and older.

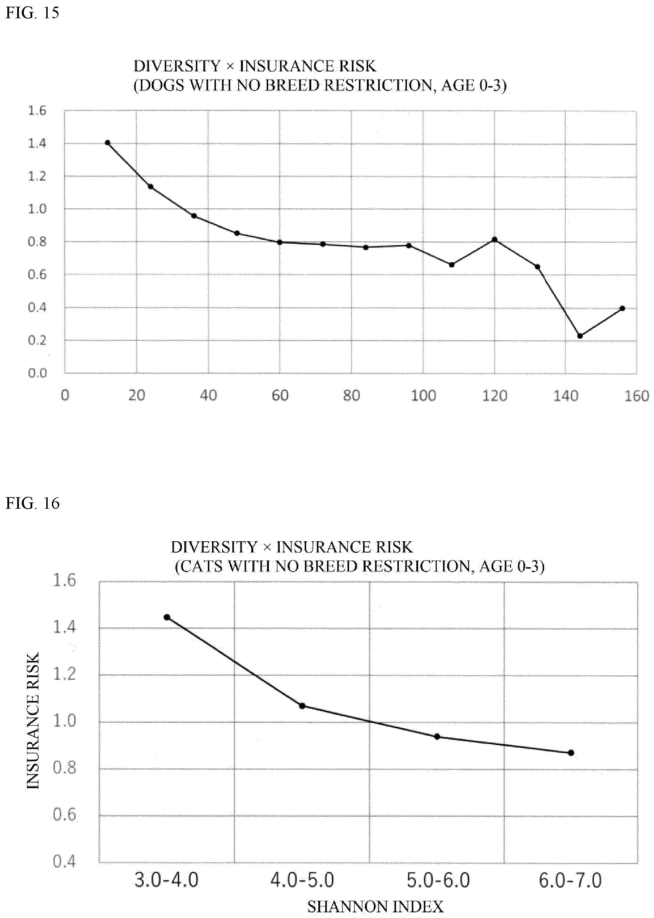

FIG. 15 also illustrates a diagram with the diversity index changed to ASV index instead of Shannon index (105,677 individuals). It can be seen that a correlation between diversity data and an insurance risk is established even when the diversity index is changed.

Furthermore, FIG. 16 illustrates a correlation chart between diversity data and an insurance risk for all cat breeds (36,312 individuals) aged 0 years and older and 3 years and younger. It can be seen that a correlation between diversity data and an insurance risk is established not only for dogs but also for other pet animals. Note that the diversity for cats is explained in detail in examples 3 and 4.

### (Simulation)

First, it is assumed that the average medical cost for dogs aged 0 to 3 years without breed restriction is 100,000 yen per year and that there is an animal insurance product of 70,000 yen (including 20,000 yen for commissions, operating expenses, and other costs) per year with 50% coverage. Here is a simulation of an insurance premium quoted for Shiba Inu A aged 2 years with diversity data of 4.3.

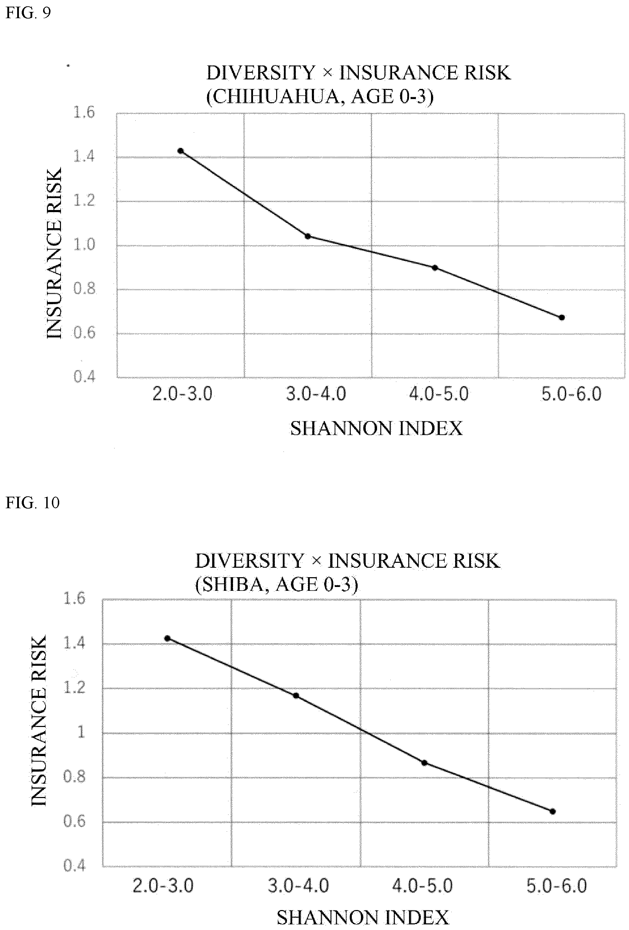

First, a correlation for "Shiba, aged 0 to 3" is set according to the age and breed (FIG. 10). Here, since the diversity data of Shiba Inu A is 4.3, the insurance risk is calculated to be about 0.9. Therefore, the medical cost for Shiba Inu A is predicted to be 90,000 yen. Since this is an insurance product with 50% coverage, the price is quoted as 45,000 yen plus 20,000 yen for commissions, operating expenses, and other costs, for a total of 65,000 yen.

### [Example 2]

The result of the research conducted by the inventors revealed that when Pomeranians that were eating only one type of commercial dry food were compared with Pomeranians that were eating two types of dry food in combination, the diversity index (Shannon index) increased by about 0.2 for the latter (from 4.2 to 4.4). In other words, by adding the information that a Pomeranian that had been eating only one type of dry food has started eating two types of dry food in combination, the diversity index can be expected to increase by about 0.2 and the future insurance risk to decrease by about 0.1 (FIG. 13). Applied to the aforementioned simulation, a medical cost and an insurance premium with a decrease by about 10,000 yen and 5,000 yen, respectively, can be presented to the user.

### [Example 3]

### (Selection of cats)

The insurance risk of approximately 110,000 individuals whose diversity indices of the intestinal microbiota were measured from fecal samples within the policy period (one year) was investigated based on insurance claims during the same period. The presence or absence of an injury or illness prior to fecal sample collection was not taken into account, so individuals with an injury or illness prior to sample collection were included.

### (DNA extraction from fecal samples and 16S RNA metagenomic sequencing analysis)

Using the same method as for dogs, composition data of the intestinal microbiota were obtained for all cat breeds aged 0 years and older and 3 years and younger, and the diversity index (Shannon index) was measured using QIIME2. The number of valid individuals was 36,312 for the Shannon index, excluding outliers.

### (Confirmation of injury/illness and insurance claim)

For all the cat breeds, the inventors investigated whether or not an insurance claim was filed within the policy period. As noted above, the presence or absence of an injury or illness prior to fecal sample collection was not considered, so individuals with injury or illness prior to sample collection were included.

### (Correlation chart)

The correlation between the diversity data and the insurance risk obtained by the above experiment is illustrated in FIG. 16. The vertical axis is the insurance risk and the horizontal axis is the diversity index.

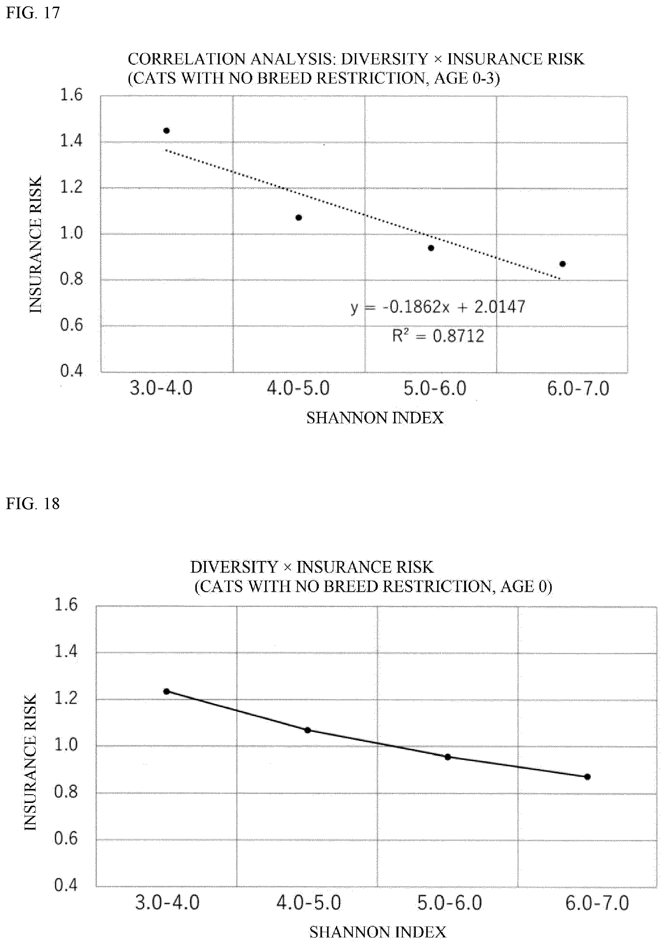

FIG. 17 illustrates the result of the correlation analysis of FIG. 16 (cats with no breed restriction, aged 0 to 3), which reveals that a correlation is established between diversity data and the insurance risk.
Correlation coefficient: -0.93 (negative correlation)
Approximate formula: y = -0.1862x + 2.0147,
Coefficient of determination: R² = 0.8712

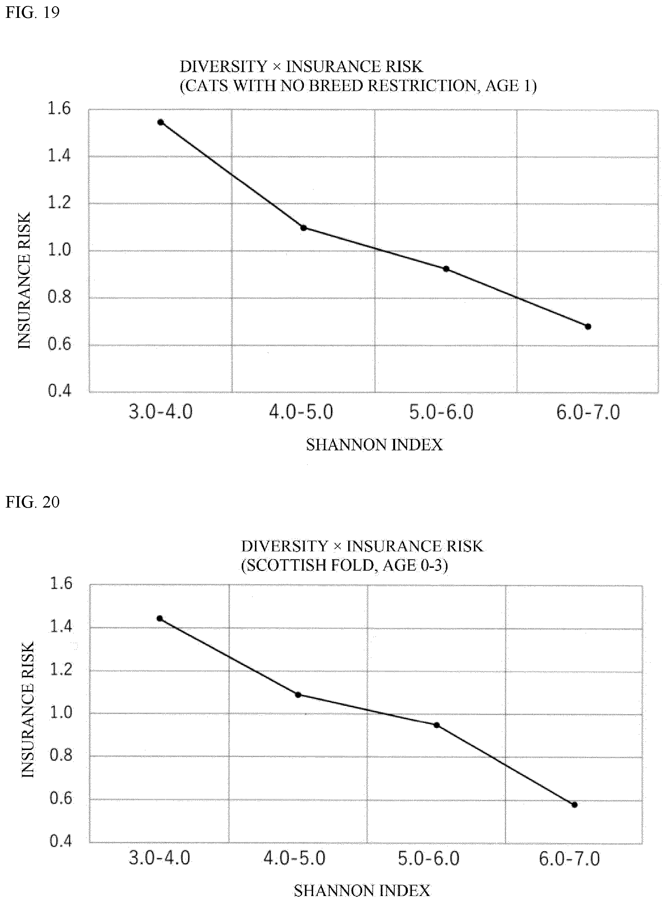

FIG. 18 illustrates a correlation between diversity data and an insurance risk for (cats with no breed restriction, age 0), and FIG. 19 illustrates a correlation for (cats with no breed restriction, age 1). The correlation is also valid not only for the total of 0 to 3 years old, but also for each age group.

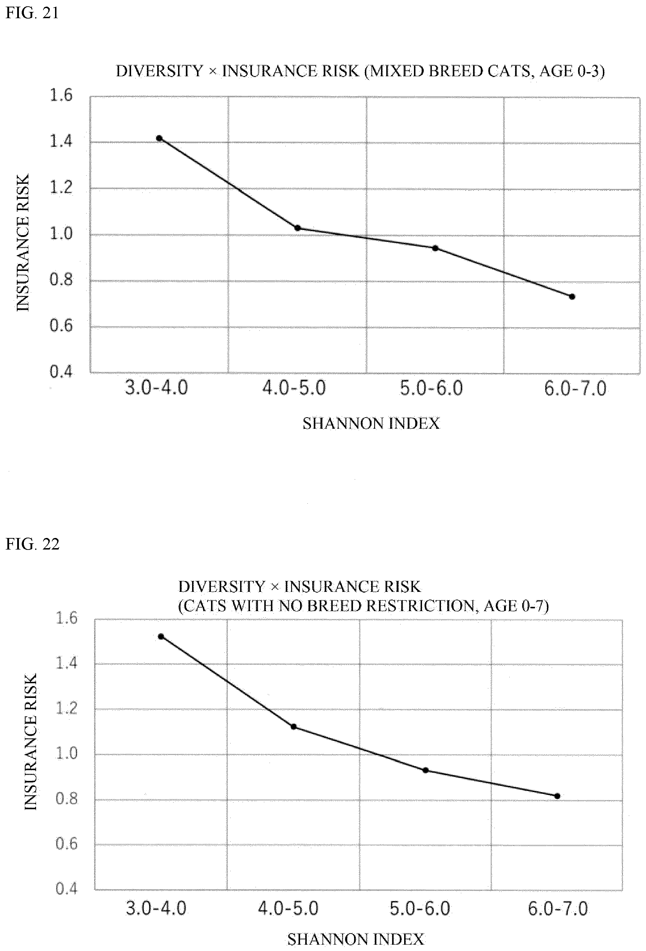

Furthermore, FIG. 20 illustrates a correlation between diversity data and an insurance risk for (Scottish Fold, aged 0 to 3) and FIG. 21 illustrates a correlation for (mixed breed cats, aged 0 to 3). It can be seen that the correlation is established even when analyzed by breed.

Furthermore, FIG. 22 illustrates a correlation chart between diversity data and an insurance risk for all cat breeds aged 0 years and older and 7 years and younger (45,400 individuals). This result reveals that there is a correlation between diversity data and insurance risk not only for aged 0 and older and aged 3 and younger, but also for aged 4 and older. It can also be seen that a correlation exists between diversity data and an insurance risk not only for dogs but also for cats.

### (Simulation)

First, it is assumed that the average medical cost for cats aged 0 to 3 years without breed restriction is 100,000 yen per year and that there is an animal insurance product of 70,000 yen (including 20,000 yen for commissions, operating expenses, and other costs) per year with 50% coverage. Here is a simulation of an insurance premium quoted for a Scottish Fold A aged 2 years with diversity data of 5.8.

First, a correlation for "Scottish Fold, aged 0 to 3" is set according to the age and breed (FIG. 20). Here, since the diversity data of Scottish Fold Ais 5.8, the insurance risk is calculated to be about 0.8. Therefore, the medical cost for Scottish Fold A is predicted to be 80,000 yen. Since this is an insurance product with 50% coverage, the price is quoted as 40,000 yen plus 20,000 yen for commissions and operating expenses and other costs, for a total of 60,000 yen.

### [Example 4]

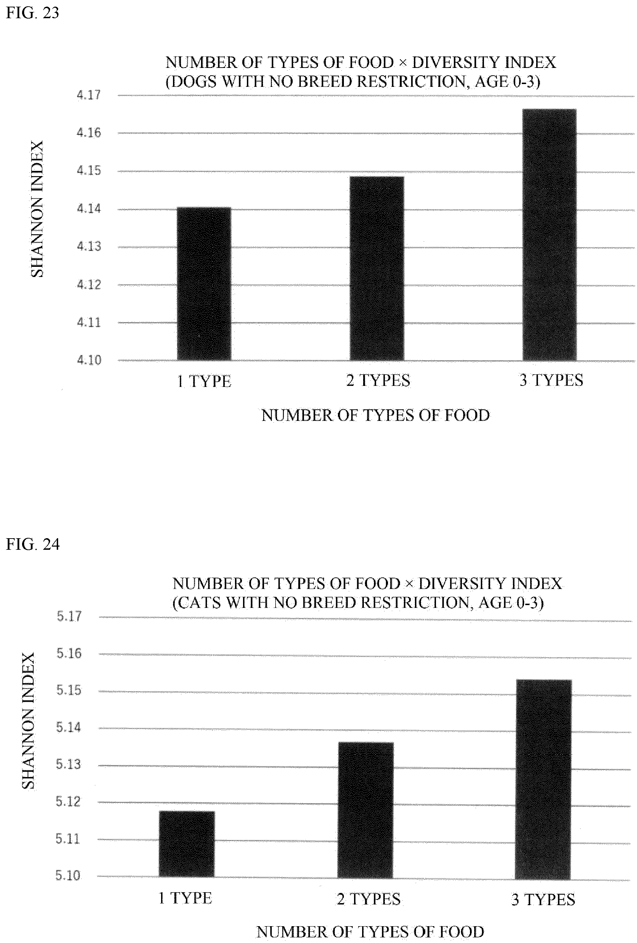

The result of the study by the inventors revealed that when dogs that were eating only one type of food were compared with dogs that were eating two or more types of food in combination, the diversity index (Shannon index) increased for the latter (FIG. 23). Similar result was obtained for cats (FIG. 24). In other words, by adding the information that a pet animal that had been eating one type of food has started eating two or more types of food in combination, the diversity index can be expected to increase according to the number of types of food it has started eating and the future insurance risk to decrease. In other words, it is possible to present to a user how much medical costs can be lowered by feeding their pet animal more than one type of food. In the present invention, information of the brand name of the food and the processing method (whether it is dry or fresh food) is not considered. This is because most of the pet foods on the market are general nutritional foods, and the influence of the brand name and processing method of the food on the diversity index is small compared to the influence of the number of types of food on the diversity index.

### [Example 4-1]

When dogs that were eating only one type of food were compared with dogs that were eating three types of food in combination, the diversity index (Shannon index) increased by about 0.025 for the latter (FIG. 23). Adding the information that Shiba Inu A in example 1, which had been eating one type of food, has started eating a combination of three types of food, the diversity of Shiba Inu A can be expected to increase from 4.3 to 4.325 and the insurance risk to decrease from about 0.9 to about 0.89 by referring to the correlation of "Shiba, aged 0 to 3" (FIG. 10). Thus, when applied to the aforementioned simulation, a predicted medical cost with a decrease by about 1,000 yen can be presented to the user.

### [Example 4-2]

The result of the study by the inventors revealed that when cats that were eating only one type of commercial dry food were compared with cats that were eating three types of dry food, the diversity index (Shannon index) increased by about 0.03 for the latter (FIG. 24). In other words, by adding the information that Scottish Fold A, which had been eating only one type of dry food, has started eating three types of food in combination, the diversity of Scottish Fold A can be expected to increase from 5.8 to 5.83 and the insurance risk to decrease from 0.8 to 0.79 by referring to the correlation of "Scottish Fold, aged 0 to 3" (FIG. 20). Thus, when applied to the aforementioned simulation, a predicted medical cost with a decrease of about 1,000 yen can be presented to the user.

### [Beauty level estimation system]

Next, a beauty level estimation system is described. The beauty level estimation system, together with the insurance premium calculation system described above, can be a component for establishing an overall health estimation system. When establishing an overall health estimation system, the beauty level estimation system and the insurance premium calculation system can be separate systems, or the same system can have two functions, beauty level estimation and insurance premium calculation.

The beauty level estimation system of the present invention includes: an acquisition unit that acquires diversity data of intestinal microbiota; and a calculation unit that calculates a beauty level of a pet animal kept by a user based on a correlation between the beauty level of the pet animal kept by the user and the diversity data. It is preferable for the beauty level estimation system of the present invention that the acquisition unit further acquires basic information of the pet animal kept by the user. Furthermore, it is preferable that the beauty level estimation system of the present invention further includes a setting unit that sets a correlation between the diversity data of the intestinal microbiota of a pet animal and a beauty level. Furthermore, it is preferred that the beauty level estimation system includes a prediction calculation unit that predicts a future beauty level of a pet animal kept by a user based on information of the food fed to the pet animal.

### [Acquisition unit]

The acquisition unit is the same as the one used in the insurance premium calculation system described above.

### [Calculation unit]

The calculation unit calculates a beauty level of a pet animal kept by a user based on the diversity data of the intestinal microbiota of the pet animal and a correlation between the diversity data of the intestinal microbiota of a pet animal and a beauty level. The calculation unit is constituted by a processor, such as a CPU, and performs processing, operation, and calculation using a formula, function, table, or software pertaining to the correlation between the diversity data of the intestinal microbiota and the beauty level.

The calculation unit may calculate a beauty level by adjusting a beauty level calculated based on basic information of a pet animal, according to a beauty variation factor derived using the diversity data of the intestinal microbiota of the pet animal. For example, a provisional beauty level is calculated based on basic information such as age and breed, and then the provisional beauty level is adjusted according to a beauty variation factor. The beauty variation factor is a variable that is set based on the correlation between the diversity data of the intestinal microbiota of the pet animal and the beauty level, for example, a value set within the range of 0.01 to 3.0, preferably 0.1 to 2.0. As a specific example, if the beauty variation factor is 0.5, a final beauty level is calculated by multiplying the provisional beauty level calculated based on the basic data (in this case, a specific numerical value) by 0.5.

### [Diversity data]

Diversity data are data related to the bacterial diversity of the intestinal microbiota of an animal and are the same as those used in the aforementioned insurance premium calculation system including the measurement method.

### [Setting unit]

The server or storage unit may include a setting unit that sets a correlation between the diversity data of the intestinal microbiota of a pet animal and a beauty level. The correlation herein refers to information indicating the correspondence between the degree of the diversity data and the degree of the beauty level. The correlation may be viewed as a model (function) with diversity data as input and beauty level as output. For example, a correlation is set by statistically processing the diversity data of a plurality of pet animals and the information of the beauty of the pet animals (for example, fur gloss). **In** principle, pet animals used to set the correlation and a pet animal kept by a user and whose beauty level is to be calculated are different individuals.

The correlation may instead be based on basic information of a pet animal kept by a user. The basic information may include age and breed. For example, the server sets a correlation by statistically processing the diversity data of the pet animals that have similar basic information to that of a pet animal kept by a user and the information on the beauty of the pet animals. The server may pre-establish a correlation for each category of basic information (for example, age, breed) and set a correlation for the category corresponding to the basic information of the pet animal kept by the user. The setting unit does not need to set a correlation each time the system runs, but may be configured in such a way that the correlation once set by the setting unit is continuously used by the calculation unit thereafter. The configuration may also be such that a correlation is set each time the data pertaining to the beauty level and diversity data are updated.

### [Overview of the beauty level estimation system]

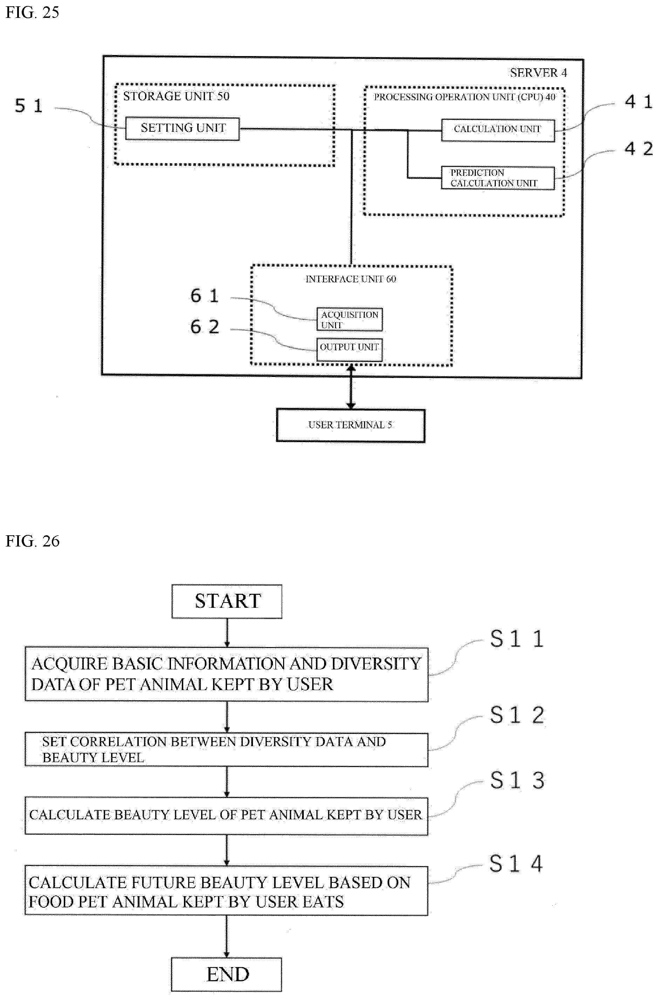

FIG. 25 is a diagram illustrating an overview of a beauty level estimation system according to an embodiment of the present disclosure. As illustrated in FIG. 25, the beauty level estimation system according to the present embodiment includes a server 4 and a user terminal 5. The server 4 and the user terminal 5 are connected via a network. The server 4 includes a processing operation unit (CPU) 40, a storage unit 50, and an interface unit 60. Users in the present invention include pet owners as well as agents, breeders, or the like. Pet animals include dogs, cats, rabbits, and birds while dogs and cats are preferred.

Furthermore, in the present embodiment, the processing operation unit (CPU) 40 includes a calculation unit 41 and a prediction calculation unit 42; the storage unit 50 includes at least a setting unit 51; and the interface unit 60 includes an acquisition unit 61 and an output unit 62. The setting unit 51 may be configured to store a formula, a function, a table or software pertaining to the correlation between the diversity data of the intestinal microbiota and the beauty level. In such a configuration, the calculation unit 41 can retrieve the formula, function, table or software stored in the setting unit 51 based on the basic information of a pet animal kept by a user, and calculate the beauty level based on the diversity data of the pet animal kept by the user. The beauty level represents the level of the appearance of the pet animal, such as whether the pet animal has good or bad fur gloss, whether the pet animal is of appropriate weight, whether the pet animal has a good or bad body shape, the condition of the skin such as skin gloss, eye discharge, and cloudiness of the eyes. Since the beauty level is a level (standard), it is preferable to output not an absolute value but a relative value compared to the average value or a class when classified. The beauty level may instead be displayed as a numerical value in the form of deviation value. The specific output of the beauty level is not restricted, and can be in the form of outputting the level for each item, such as fur gloss, appropriate weight, and body shape, or calculating the overall score by summing the level for each item or calculating the average value, and then outputting the evaluation value in accordance with the overall score. The output format can be a method of displaying a relative numerical value such as a deviation value as described above, a method of displaying a classification such as "good," "normal," or "bad," or a method of displaying a value relative to the average value such as "+1," "+2," "0," or "-1."

Here, the calculation unit 41 calculates a beauty level of a pet animal kept by a user based on the diversity data of the pet animal kept by the user and the correlation mentioned above. The embodiment of FIG. 25 further includes a prediction calculation unit 42. The prediction calculation unit 42 predicts a future beauty level of the pet animal kept by the user based on the information of the food fed to the pet animal. For example, a beauty level is calculated by adjusting a beauty level once calculated by the calculation unit 41, based on the food information.

The setting unit 51 also sets a correlation between the diversity data of the intestinal microbiota of a pet animal and a beauty level. As described above, a formula or function pertaining to the correlation may be stored in advance, or the setting unit 51 may be configured to set a formula or function based on a new correlation.

Furthermore, the acquisition unit 61 acquires the diversity data of the intestinal microbiota and, preferably, further acquires basic information of the pet animal kept by the user, and the output unit 62 sends the beauty level and other information calculated by the calculation unit 41 to the user.

### [Beauty level estimation method]

FIG. 26 illustrates an overview of a beauty level estimation method according to an embodiment of the present disclosure. As illustrated in FIG. 26, the beauty level estimation method according to the present embodiment includes the following steps in the following order: Step S11 for acquiring basic information of a pet animal kept by a user and diversity data of the intestinal microbiota; Step S12 for setting a correlation between the diversity data of the intestinal microbiota of a pet animal and a beauty level; and Step S13 for calculating a beauty level of the pet animal kept by the user based on the diversity data of the pet animal kept by the user and the correlation.

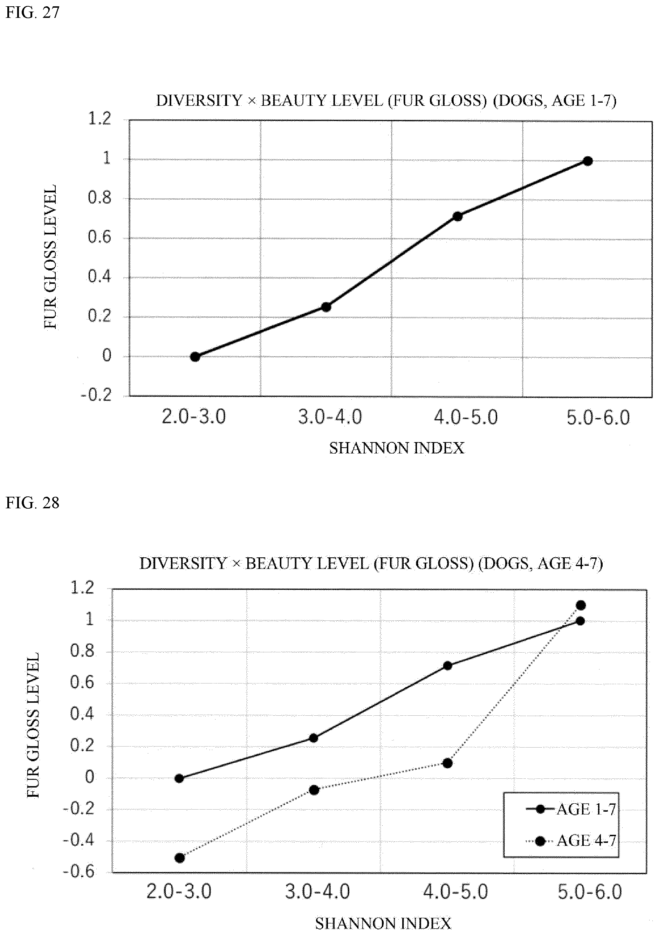

Here, it is preferable to further include a step for acquiring basic information of the pet animal kept by the user, and for adjusting the beauty level based on the basic information of the pet animal kept by the user. By taking into account the basic information of the pet animal kept by the user, a beauty level more appropriate for the pet animal can be calculated. For example, by adding the information that the age of the pet animal is 5 years old, the correlation illustrated in FIG. 28 can be adopted instead of FIG. 27, which enables a more accurate estimation of the fur gloss level of the pet animal.

Furthermore, the system may also include Step S14 for predicting a future beauty level of the pet animal kept by the user based on information of the food that the pet animal kept by the user eats. As explained in the aforementioned insurance premium calculation system, the future beauty level can be calculated by taking into account the information of the food, since the diversity of the pet animal increases depending on the food the pet animal eats.

### [Mode of using a learned model]

The beauty level estimation system of the present invention may employ a learned model. That is, the beauty level estimation system according to another embodiment of the present invention includes: an acquisition unit that acquires the diversity data of the intestinal microbiota of a pet animal kept by a user; and a calculation unit that calculates a beauty level of the pet animal from the diversity data of the intestinal bacteria acquired by the acquisition unit using a learned model. The beauty level estimation system is characterized in that the learned model is a learned model that has learned the relationship between the diversity data of the intestinal microbiota of a pet animal and the beauty level of the pet animal.

The learned model can be generated, for example, by supervised or unsupervised learning. Training data in the case of supervised learning include, for example, diversity data of the intestinal microbiota of an animal and data or labels on the beauty level of the animal.

The method for identifying a beauty level to be used as training data may be, for example, identifying a beauty level based on questionnaires to pet owners, descriptions written on pet insurance applications, questionnaires to trimmers and other professionals.

The learned model is preferably artificial intelligence (AI). Artificial intelligence (AI) refers to software or a system whereby a computer mimics the intellectual tasks performed by the human brain, specifically, a computer program that understands a natural language used by humans, performs logical reasoning, and learns from experience. The artificial intelligence can be general-purpose or specialized, and can be any of the known types, such as logistic regression, decision trees, k-means, multilayer perceptrons, recurrent neural networks, deep neural networks, and convolutional neural networks. A publicly available software can be used.

To generate a learned model, the artificial intelligence is trained. Learning can be either machine learning or deep learning, but deep learning is preferred. Deep learning is an advanced form of machine learning and is characterized by its ability to automatically find feature quantities.

The learning method for generating a learned model is not restricted and can use publicly available software or libraries. The learning method may be transfer learning. For example, DIGITS (the Deep Learning GPU Training System) published by NVIDIA can be used. Also, for example, ResNet, MobileNet, or EfficientNet can be used as artificial intelligence (neural networks), and machine learning libraries (Deep Learning libraries) such as Pytorch can be used to generate a learned model by transfer learning. Other known support vector machines published in, for example, "Introduction to Support Vector Machines" (Kyoritsu Shuppan) may also be used.

As illustrated in the examples, the inventors have found that there is a correlation between the diversity data of the intestinal microbiota of an animal and the beauty level of the animal. Therefore, if the diversity data of the intestinal microbiota of an animal and the beauty level of the animal are used as training data for learning, a learned model that has learned the relationship between the diversity data of the intestinal microbiota of a pet animal and the beauty level of the pet animal, especially, a learned model in which the input is the diversity data of the intestinal microbiota of the pet animal and the output is the beauty level of the pet animal, is obtained.

### [Overall health estimation system]

Furthermore, the present invention provides an overall health estimation system that calculates an overall health level based on the aforementioned beauty level and insurance risk. Here, the indicator related to the overall health level can be set by a user as desired. For example, since the beauty level is an indicator to determine whether a pet animal is unwell (not sick but not healthy) while the insurance risk is an indicator reflecting a manifested illness, a user can adjust the weighting of each indicator before adding them together. Specifically, an overall health level index may be the insurance risk minus a value obtained by multiplying the beauty level by 0.1.

### [Beauty level estimation method]

The beauty level estimation method of the present invention includes the steps, in the following order, of: acquiring the diversity data of the intestinal microbiota of a pet animal; and calculating, by a computer, a beauty level of the pet animal kept by a user based on the diversity data and a correlation between the diversity data of the intestinal microbiota of a pet animal and a beauty level. Furthermore, it is preferable to further include the step of acquiring basic information of the pet animal kept by the user, and that the basic information of the pet animal includes at least breed and age.

Moreover, the beauty level estimation method of the present invention preferably further includes the step of adjusting, based on information of food that the pet animal kept by the user eats, a beauty level derived in the step of calculating a beauty level of the pet animal kept by the user based on the diversity data of the pet animal kept by the user and the correlation between the diversity data of the intestinal microbiota of the pet animal and the beauty level. The diversity data, beauty level, and correlation are the same as in the beauty level estimation system described above.

Next, the technical features of the present invention are explained in detail with reference to an example.

### [Example 5]

### [Calculation of beauty level based on diversity data]

The following describes an example of the beauty level calculation method based on diversity data with reference to FIG. 27. FIGS. 27 to 29 are graphical illustrations illustrating a correlation between diversity data of intestinal microbiota and a beauty level, for which the inventors collected and plotted data.

FIG. 27 illustrates a correlation between a fur gloss level, a kind of beauty level, and a diversity index for dogs aged 1 to 7 years without breed restriction (134,813 dogs), where the vertical axis is the fur gloss level and the horizontal axis is the diversity index (Shannon index). As described in FIG. 27, it can be seen that a correlation exists between the data on diversity and the fur gloss level.

The following explains how the fur gloss level was set. First, for statistical analysis, the diversity index was divided into four categories ((1) 2.0 or more and less than 3.0, (2) 3.0 or more and less than 4.0, (3) 4.0 or more and less than 5.0, and (4) 5.0 or more and less than 6.0). For each of these categories, a person with expertise (a trimmer) judged the fur gloss level based on the gloss and luster of the fur. More specifically, among the 1 to 7 years old dogs with no breed restriction, the average fur gloss in the group with a diversity index of 2.0 or more and less than 3.0 was set to "0 (middle standard)," the average fur gloss in the group with a diversity index of 5.0 or more and less than 6.0 was set to "1 (superior standard)." Among the 7 year old dogs with no breed restriction, the average fur gloss in the group with a diversity index of 2.0 or more and less than 3.0 was set to "-1" (poor standard). The evaluation was performed according to such criteria.

FIG. 28 illustrates a correlation between a fur gloss level and a diversity index of dogs aged 4 to 7 years without breed restriction (48,563 dogs), and data of dogs aged 1 to 7 years without breed restriction are also included for comparison. As illustrated in FIG. 28, the correlation can be confirmed even if the age group changes, and, for the older age groups, the change in fur gloss with the change in diversity index is significant.

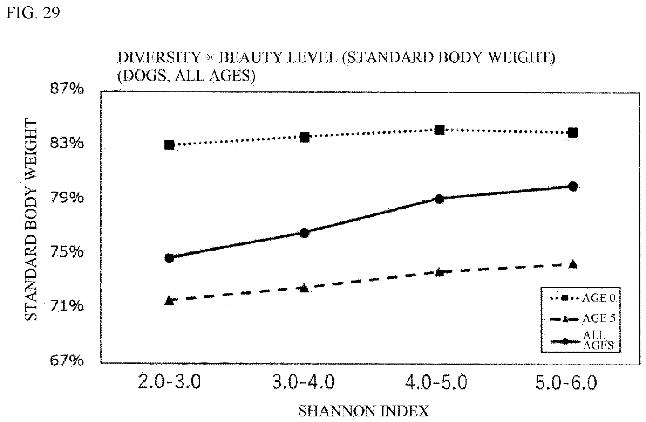

The beauty level is not limited to fur gloss alone. For example, FIG. 29 illustrates a correlation between a standard body weight (a range neither thin nor fat) and a diversity index where the vertical axis is the percentage of individuals with standard body weight (%) and the horizontal axis is the diversity index (Shannon index). As illustrated in FIG. 29, a correlation is established not only for fur gloss but also for standard body weight, indicating that standard body weight can be used as an indicator of beauty level.

The present invention may also set an indicator that combines the standard body weight and fur gloss.

## Claims

1. An insurance premium calculation system comprising:
an acquisition unit that acquires diversity data of intestinal microbiota of a pet animal kept by a user; and
a calculation unit that calculates an insurance risk of the pet animal kept by the user based on the diversity data of the intestinal microbiota and a correlation between diversity data of intestinal microbiota of a pet animal and an insurance risk.

2. The insurance premium calculation system according to claim 1, wherein the acquisition unit further acquires basic information of the pet animal kept by the user.

3. The insurance premium calculation system according to claim 1 or 2, further comprising a setting unit that sets a correlation between diversity data of intestinal microbiota of a pet animal and an insurance risk,
wherein the calculation unit calculates an insurance risk of the pet animal kept by the user based on the diversity data of the intestinal microbiota and the correlation set by the setting unit.

4. The insurance premium calculation system according to claim 1, wherein the correlation between the diversity data of the intestinal microbiota of the pet animal and the insurance risk is a negative correlation.

5. The insurance premium calculation system according to claim 1, wherein the diversity data of the intestinal microbiota of the pet animal is represented by a Shannon index.

6. The insurance premium calculation system according to claim 2, wherein the calculation unit calculates an insurance premium by adjusting an insurance premium calculated based on the basic information of the pet animal, according to an insurance risk derived using the diversity data of the intestinal microbiota of the pet animal.

7. The insurance premium calculation system according to claim 2, wherein the calculation unit calculates an insurance premium based on a value obtained by adjusting a predicted medical cost calculated based on the basic information of the pet animal, according to an insurance risk derived using the diversity data of the intestinal microbiota.

8. The insurance premium calculation system according to claim 2, wherein the calculation unit calculates an insurance premium based on a value obtained by adjusting a predicted medical cost calculated based on the basic information of the pet animal by a factor of 0.5 to 2.0, according to an insurance risk derived using the diversity data of the intestinal microbiota.

9. The insurance premium calculation system according to claim 2, wherein the basic information of the pet animal is at least breed and age.

10. The insurance premium calculation system according to claim 1 or 2, further comprising a prediction calculation unit that adjusts an insurance risk calculated by the calculation unit, based on information of food consumed by the pet animal kept by the user.

11. An insurance premium calculation method comprising, in the following order, the steps of:
acquiring diversity data of intestinal microbiota; and
calculating, by a computer, an insurance risk of a pet animal kept by a user based on the diversity data of the pet animal kept by the user and a correlation.

12. The insurance premium calculation method according to claim 11, further comprising the step of acquiring basic information of the pet animal kept by the user.

13. The insurance premium calculation method according to claim 12, wherein the basic information of the pet animal is at least breed and age.

14. The insurance premium calculation method according to claim 11 or 12, further comprising the step of adjusting, based on information of food that the pet animal kept by the user eats, an insurance risk derived in the step of calculating the insurance risk of the pet animal kept by the user based on the diversity data of the pet animal kept by the user and the correlation.

15. A financial burden predicting method comprising, in the following order, the steps of:
acquiring basic information of a pet animal kept by a user and diversity data of intestinal microbiota;
setting a correlation between diversity data of intestinal microbiota of a pet animal and a burden risk; and
calculating, by a computer, a burden risk of the pet animal kept by the user based on the diversity data of the pet animal kept by the user and the correlation.

16. A beauty level estimation system comprising:
an acquisition unit that acquires diversity data of intestinal microbiota of a pet animal kept by a user; and
a calculation unit that calculates a beauty level of the pet animal kept by the user based on the diversity data of the intestinal microbiota of the pet animal and a correlation between diversity data of intestinal microbiota of a pet animal and a beauty level.

17. The beauty level estimation system according to claim 16, wherein the acquisition unit further acquires basic information of the pet animal kept by the user.

18. The beauty level estimation system according to claim 16 or 17, wherein the beauty level is a level related to fur gloss or a level related to a body shape.

19. The beauty level estimation system according to claim 16, wherein the diversity data of the intestinal microbiota of the pet animal is represented by a Shannon Index.

20. The beauty level estimation system according to claim 17, wherein the calculation unit calculates a beauty level by adjusting a beauty level calculated based on the basic information of the pet animal according to a beauty variation factor derived using the diversity data of the intestinal microbiota of the pet animal.

21. The beauty level estimation system according to claim 17, wherein the basic information of the pet animal is at least breed and age.

22. A beauty level estimation system, comprising:
an acquisition unit that acquires diversity data of intestinal microbiota of a pet animal kept by a user; and
a calculation unit that calculates a beauty level of the pet animal from the diversity data of the intestinal bacteria acquired by the acquisition unit using a learned model,
wherein the learned model is a learned model that has learned a relationship between diversity data of intestinal microbiota of a pet animal and a beauty level of the pet animal.

23. An overall health estimation system comprising:
an acquisition unit that acquires diversity data of intestinal microbiota of a pet animal kept by a user;
a calculation unit that calculates a beauty level of the pet animal kept by the user based on the diversity data of the intestinal microbiota and a correlation between diversity data of intestinal microbiota of a pet animal and a beauty level;
a risk calculation unit that calculates an insurance risk of the pet animal kept by the user based on the diversity data of the intestinal microbiota and a correlation between diversity data of intestinal microbiota of a pet animal and an insurance risk; and
a health level calculation unit that calculates a health level based on the beauty level and the insurance risk.

24. A beauty level estimation method comprising, in the following order, the steps of:
acquiring diversity data of intestinal microbiota of a pet animal; and
calculating, by a computer, a beauty level of the pet animal kept by a user based on the diversity data and a correlation between diversity data of intestinal microbiota of a pet animal and a beauty level.

25. The beauty level estimation method according to claim 24, further comprising the step of acquiring basic information of the pet animal kept by the user.

26. The beauty level estimation method according to claim 25, wherein the basic information of the pet animal is at least breed and age.

27. The beauty level estimation method according to claim 25 or 26, further comprising the step of adjusting, based on information of food that the pet kept by the user eats, a beauty level derived in the step of calculating the beauty level of the pet animal kept by the user based on the diversity data of the pet animal kept by the user and the correlation between the diversity data of the intestinal microbiota of the pet animal and the beauty level.
